# EUROPEAN PATENT APPLICATION

(11) **EP 2 514 348 A2**
(43) Date of publication of application: **24.10.2012**
(21) Application number: 10837866.2
(22) Date of filing: 15.12.2010
(51) Int. Cl.: A47L 9/28, A47L 5/14, A47L 7/04

(54) **REFLUX-TYPE EXHAUST ROBOT CLEANING DEVICE**

(30) Priority: 15.12.2009 KR 20090124460
(71) Applicant: Hanwool Robotics Corporation, Wonmi-Gu Bucheon-City, Gyunggi-Do 420-734 (KR)
(72) Inventor: KIM, Byung Soo, Gyeonggi-Do, 446-913 (KR); LEE, Byung Soo, Incheon 406-754 (KR); LEE, Nam Su, Bucheon-si Gyeonggi-do 420-830 (KR); LEE, Dong Hoon, Goheung-gun Jeollanam-do 548-873 (KR)
(74) Representative: Dossmann, Gérard
(86) International application number: PCT/KR2010/008967
(87) International publication number: WO 2011/074870

(57) **Abstract**

Disclosed is an exhaust reflux type cleaning robot. In the cleaning robot, foreign substances along with air are sucked into a suction unit by a suction motor, and the foreign substances are collected in a dust collecting unit while the air from which the foreign substances have been removed is discharged to the outside via the suction motor. The cleaning robot includes an exhaust reflux unit which has the suction motor and is provided with left and right air passages disposed on opposite sides of the suction motor, a discharge nozzle unit which is connected to the left and right air passages, a suction unit which is configured such that the discharge nozzle unit is disposed in front of the suction unit, and a sterilization anion generating unit which generates sterilization anions to sterilize air discharged via the suction motor.

## Description

### Technical Field

The present invention relates, in general, to exhaust reflux type cleaning robots and, more particularly, to an exhaust reflux type cleaning robot which not only has a cleaning function but also provides sterilization anions to exhaust air, thus sterilizing and purifying circulation air.

### Background Art

Generally, cleaning robots move in target areas by themselves without being manipulated by users and suck foreign substances, such as dust, etc., off of floor surfaces, thus automatically cleaning the target areas. If the power of a battery is low, a cleaning robot moves by itself to a recharging position so as to recharge the battery and then returns to a location where it had been cleaning and continues cleaning.

Such a cleaning robot moves by itself in a target cleaning area in a predetermined running pattern and removes foreign substances from a surface to be cleaned. However, if foreign substances adhere to the surface to be cleaned or a carpet, the cleaning robot may not reliably remove such foreign substances while moving in the target cleaning area in the running pattern.

Furthermore, given a location where the cleaning robot is used or the mobility thereof, the size and weigh of the cleaning robot are restricted, in other words, the cleaning robot should be reduced in size and weight. Therefore, a comparatively large capacity suction motor cannot be used, resulting in a reduction in the suction force of the cleaning robot, thus making it difficult to reliably remove foreign substances from the surface to be cleaned.

The above problems are more markedly induced in vacuum cleaning robots. Because of these problems, a cleaning robot may drag foreign substances when it moves around, rather than sucking them, thus causing a problem of the cleaning surface being increased.

To overcome the problem of the low suction force of a small suction motor, a suction brushing method which combines a vacuum suction method and a brushing method was proposed. According to the suction brushing method, a brush moves foreign substances upwards into the cleaning robot, and the cleaning robot sucks the rising foreign substances using the force of vacuum suction. Therefore, although foreign substances that are brought into contact with the brush can be easily removed from the cleaning surface, other foreign substances that have adhered to the cleaning surface must be removed only by the vacuum suction method. Therefore, foreign substances may not be reliably removed. Particularly, in the case of the suction brushing method, because a suction port is disposed above the brush, the suction force thereof drops even more, thus causing foreign substances to remain on the cleaning surface if the foreign substances have not been removed by the brush.

As such, the suction brushing method uses a brush, supplementing for the weakness of the vacuum suction method. However, this cannot be a fundamental solution to the problem of residual foreign substances. In addition, the brushing method requires a separate device for brushing, thus increasing the production cost, and making maintenance and repair of elements difficult.

In the conventional cleaning robots, air and dust are sucked together into a suction port. The dust is collected in a dust collecting unit, and air from which dust has been removed is exhausted out of an exhaust port after being used to cool the suction motor. Such exhaust air blows dust that has been around the cleaning robot, thus causing the dust to be scattered into the air.

Furthermore, the conventional cleaning robots merely run around, suck foreign substances from a floor surface (a cleaning surface) and remove dust or dirt. However, a lot of dust still remains in the air that is discharged after the foreign substances have been removed therefrom, thus affecting the respiratory system of a user.

### Disclosure

### Technical Problem

Accordingly, the present invention has been made keeping in mind the above problems occurring in the prior art, and an object of the present invention is to provide an exhaust reflux type cleaning robot which is provided with a sterilization anion generator so as to sterilize and purify exhaust air that is discharged onto a cleaning surface and upwards from an upper surface of the cleaning robot.

Another object of the present invention is to provide an exhaust reflux type cleaning robot which is configured such that anions are discharged using exhaust reflux in a variety of directions, as well as being discharged towards the cleaning surface, and then sucked again into the cleaning robot, thus providing an air purification function.

### Technical Solution

In order to accomplish the above objects, the present invention provides an exhaust reflux type cleaning robot, in which a foreign substance of a cleaning surface along with air is sucked into a suction unit by a suction motor, and the sucked foreign substance is collected in a dust collecting unit while the air from which the foreign substance has been removed is discharged to an outside via the suction motor, the exhaust reflux type cleaning robot including: an exhaust reflux unit in which the suction motor is installed, the exhaust reflux unit being provided with left and right air passages disposed on respective opposite sides of the suction motor; a discharge nozzle unit connected to ends of the left and right air passages; a suction unit configured such that the discharge nozzle unit is disposed in front of the suction unit; and a sterilization anion generating unit generating sterilization anions to sterilize air discharged via the suction motor, wherein the discharge nozzle unit comprises a plurality of discharge ports oriented towards a floor surface and in a direction opposite to the floor surface, and the suction unit comprises a plurality of suction ports oriented towards the floor surface and in the direction opposite to the floor surface.

The discharge nozzle unit may include a first discharge nozzle provided in a lower surface of the cleaning robot, and a second discharge nozzle provided in an upper surface of the cleaning robot. The suction unit may include a first suction port provided in the lower surface of the cleaning robot, and a second suction port provided in the upper surface of the cleaning robot.

Anions discharged from the discharge nozzle unit towards the cleaning surface and air around the cleaning robot may be sucked by the suction unit and re-discharged, along with the anions generated from the sterilization anion generating unit, out of the upper and lower surfaces of the cleaning robot.

### Advantageous Effects

In an exhaust reflux type cleaning robot according to the present invention, not only air that is discharged onto a cleaning surface but air that is discharged upwards from an upper surface of the cleaning robot can also be sterilized and purified.

Furthermore, in the exhaust reflux type cleaning robot according to the present invention, anions are discharged using exhaust reflux towards the cleaning surface and into the air around the cleaning robot and then sucked again into the cleaning robot. Therefore, an air purification function can be enhanced.

### Description of Drawings

Fig. 1 is a view showing the construction of an exhaust reflux type cleaning robot, according to an embodiment of the present invention; and
Fig. 2 is a view showing the construction of an exhaust reflux unit of the exhaust reflux type cleaning robot according to the embodiment of the present invention.

### Best Mode

Hereinafter, although a preferred embodiment of the present invention will be described in detail with reference to the attached drawings, the present invention is not limited to the preferred embodiment. Reference should be made to the drawings, in which the same reference numerals are used throughout the different drawings to designate the same or similar components. The techniques that are well-known to those skilled in this art or the repetition of the same description will be omitted.

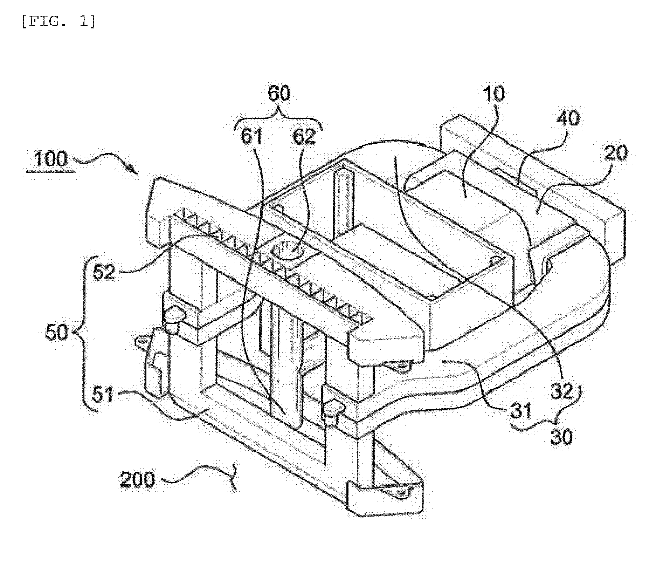

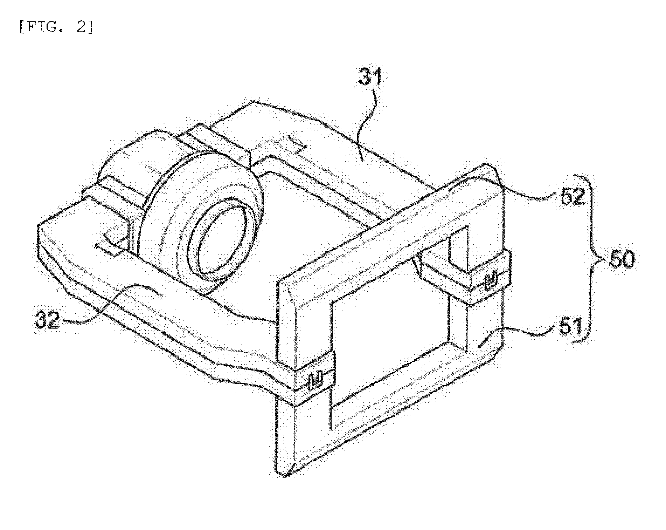

Fig. 1 is a view showing the construction of an exhaust reflux type cleaning robot, according to the present invention. Fig. 2 is a view showing the construction of an exhaust reflux unit of the cleaning robot.

The exhaust reflux type cleaning robot 100 is configured such that foreign substances along with air are sucked by a suction motor 10 from a cleaning surface 200, and the sucked foreign substances are collected in a dust collecting unit 20 while air from which the foreign substances have been removed is exhausted out of the cleaning robot via the suction motor 10.

The exhaust reflux type cleaning robot 100 includes an exhaust reflux unit 30, a sterilization anion generating unit 40, a discharge nozzle unit 50 and a suction unit 60.

The exhaust reflux unit 30 is installed in the cleaning robot 100 and functions to move air, from which foreign substances have been removed by the dust collecting unit 20, to the discharge nozzle unit 50. The exhaust reflux unit 30 includes a housing which is coupled to the dust collecting unit 20 and has the suction motor 10 therein, and left and right air passages 31 and 32 which are connected at first ends thereof to respective opposite sides of the housing while second ends thereof are connected to the discharge nozzle unit 50. The exhaust reflux unit 30 having the above-mentioned construction has been explained in detail in Korean Patent Registration No. 0869822 which was filed by the applicant of the present invention. Therefore, further explanation is deemed unnecessary.

The sterilization anion generating unit 40 is disposed between the housing of the exhaust reflux unit and the left and right air passages to provide sterilization anions into the left and right air passages 31 and 32.

Sterilization anions discharged from the sterilization anion generating unit 40 sterilize air that is being exhausted after foreign substances have been removed from the air by the dust collecting unit 20. Simultaneously, the sterilization anions are moved along the left and right air passages 31 and 32 by the flow of the exhaust air and then discharged by the discharge nozzle unit 50 towards the cleaning surface and upwards from the cleaning robot.

The discharge nozzle unit 50 uniformly discharges exhaust air and sterilization anions, which are transferred from the exhaust reflux unit 30, towards the cleaning surface and upwards from the cleaning robot. The discharge nozzle unit 50 is installed on the second ends of the left and right air passages 31 and 32 in the cleaning robot 100 such that the discharge nozzle unit 50 is oriented towards the cleaning surface and upwards from the cleaning robot.

The discharge nozzle unit 50 is disposed ahead of the suction unit 60. That is, suction ports 61 and 62 are provided in the suction unit 60 behind the discharge nozzle unit 50 with respect to the direction in which the cleaning robot moves.

Furthermore, a diffusion prevention bar may be provided on the suction unit 60 behind the support ports 61 and 62 to prevent exhaust air and sterilization anions from being undesirably diffused when they are exhausted, or wheels may be provided on the suction unit 60 to provide mobility.

The discharge nozzle unit 50 will now be explained in more detail. The discharge nozzle unit 50 includes a lower discharge nozzle 51 and an upper discharge nozzle 52 which are respectively provided under a lower surface and on an upper surface of the cleaning robot 100.

The lower discharge nozzle 51 is configured such that it is oriented towards the cleaning surface (200; the floor surface) from the left and right air passages 31 and 32 so that foreign substances that have been on the cleaning surface 200 can be blown up and easily sucked into the lower suction port 61.

The upper discharge nozzle 52 protrudes upwards from the left and right air passages 31 and 32 towards the upper surface of the cleaning robot 100. Anions generated from the sterilization anion generating unit 40 are discharged by the upper discharge nozzle 52 into the air, thus providing the effect of air purification.

Typically, the density of pollutants distributed in a house is highest within a range from the floor surface to a height of 30 cm. In the present invention, the upper discharge nozzle 52 discharges air that contains sterilization anions upwards from the cleaning robot 100. Thus, the effect of the air purification can be enhanced.

Because it may be necessary for the air discharge force of the lower discharge nozzle 51 to be greater than that of the upper discharge nozzle 52, the diameter of an air flow passage of the upper discharge nozzle 52 is preferably is less than that of the lower discharge nozzle 51 so that the amount of air that is supplied to the lower discharge nozzle 51 is greater than that of the upper discharge nozzle 52.

Furthermore, a valve may be provided at a predetermined position on the air flow passage of the upper discharge nozzle 52 to control the area of the air flow passage of the upper discharge nozzle 52.

The suction unit 60 will be explained in more detail. The suction unit 60 includes a lower suction port 61 and an upper suction port 62 which are respectively provided under the lower surface and on the upper surface of the cleaning robot 100.

The lower suction port 61 is oriented towards the cleaning surface 200. The upper suction port 62 protrudes upwards from the cleaning robot 100. The lower suction port 61 and the upper suction port 62 combine with each other and are connected to the suction motor 10.

The upper suction port 62 is disposed adjacent to the discharge nozzle 52. The upper suction port 62 sucks polluted air that is above the cleaning robot 100 and transfers it to the exhaust reflux unit 30 and the sterilization anion generating unit 40 to purify it.

In this embodiment, although the upper discharge nozzle 52 and the suction port 62 have been illustrated as being disposed in the upper portion of the cleaning robot 100, the present invention is not limited to this structure. For example, the upper discharge nozzle 52 or the upper suction port 62 may be disposed on a side, front or rear surface of the cleaning robot 100. The upper discharge nozzle 52 or the upper suction port 62 may branch off a plurality of parts from the left and right air passages 31 and 32.

In a cleaning pattern wherein the cleaning robot 100 moves and conducts the cleaning operation, dust, foreign substances and air are sucked into the suction ports 61 and 62 of the suction unit 60 and then transferred into the dust collecting unit 20. Dust and foreign substances are collected in the dust collecting unit 20, and air from which dust has been removed is transferred into the exhaust reflux unit 30 provided with the suction motor 10. Air which has been transferred into the exhaust reflux unit 30 is sterilized by anions generated from the sterilization anion generating unit 40. The sterilized air and sterilization anions are discharged by the discharge nozzle unit 50 towards the cleaning surface 200 and to the air around the cleaning robot 100.

As such, when exhaust air and sterilization anions are discharged towards the cleaning surface and to the air, foreign substances which were adhered to the cleaning surface are detached therefrom and blown up by strong discharge force of the exhaust air and, simultaneously, air around the cleaning surface 200 and the cleaning robot 100 is sterilized or purified by the discharged sterilization anions. The blown foreign substances and sterilized air are sucked again into the dust collecting unit 20 through the suction ports 61 and 62 by the driving power of the suction motor, and air recirculates.

Furthermore, anions which have been discharged from the discharge nozzle unit 50 towards the cleaning surface 200 and to the air around the cleaning robot 100 are sucked by the suction unit 60 and then re-discharged, along with anions generated from the sterilization anion generating unit 40, towards the cleaning surface and to the air, thus enhancing the sterilization and purification performance.

Although the preferred embodiment of the present invention has been disclosed for illustrative purposes, those skilled in the art will appreciate that various modifications, additions and substitutions are possible, without departing from the scope and spirit of the invention as disclosed in the accompanying claims.

## Claims

1. An exhaust reflux type cleaning robot, in which a foreign substance of a cleaning surface along with air is sucked into a suction unit by a suction motor, and the sucked foreign substance is collected in a dust collecting unit while the air from which the foreign substance has been removed is discharged to an outside via the suction motor, the exhaust reflux type cleaning robot comprising:
an exhaust reflux unit in which the suction motor is installed, the exhaust reflux unit being provided with left and right air passages disposed on respective opposite sides of the suction motor;
a discharge nozzle unit connected to ends of the left and right air passages;
a suction unit configured such that the discharge nozzle unit is disposed in front of the suction unit; and
a sterilization anion generating unit generating sterilization anions to sterilize air discharged via the suction motor,
wherein the discharge nozzle unit comprises a plurality of discharge ports oriented towards a floor surface and in a direction opposite to the floor surface, and the suction unit comprises a plurality of suction ports oriented towards the floor surface and in the direction opposite to the floor surface.

2. The exhaust reflux type cleaning robot according to claim 1, wherein
the discharge nozzle unit comprises a first discharge nozzle provided in a lower surface of the cleaning robot, and a second discharge nozzle provided in an upper surface of the cleaning robot, and
the suction unit comprises a first suction port provided in the lower surface of the cleaning robot, and a second suction port provided in the upper surface of the cleaning robot.

3. The exhaust reflux type cleaning robot according to claim 1 or 2, wherein anions discharged from the discharge nozzle unit towards the cleaning surface and air around the cleaning robot are sucked by the suction unit and re-discharged, along with the anions generated from the sterilization anion generating unit, out of the upper and lower surfaces of the cleaning robot.
